# EUROPEAN PATENT SPECIFICATION

(11) **EP 2 305 444 B1**
(45) Date of publication and mention of the grant of the patent: **16.07.2014**
(21) Application number: 10188943.4
(22) Date of filing: 22.01.2007
(51) Int. Cl.: B29C 45/14, B29C 39/02, A61M 16/06, B29C 33/40, B29C 69/00, A62B 18/02, B65B 7/16

(54) **Method for manufacturing a hollow structure**
Verfahren zur Herstellung eines Hohlkörpers
Procédé de fabrication d'une structure creuse

(43) Date of publication of application: 06.04.2011
(62) Divisional of application: 07100927.8
(73) Proprietor: ResMed R&D Germany GmbH, 82152 Martinsried (DE)
(72) Inventor: Lang, Bernd, 82166 Gräfelfing (DE); Burz, Sebastian, 87656 Germaringen (DE); Nibu, Adel, 81241 München (DE); Biener, Achim, 85445 Aufkirchen (DE); Nickol, Johannes, 80636 München (DE)
(74) Representative: Vossius & Partner

(56) References cited:
- EP-A1- 0 289 750
- FR-A- 1 200 522
- GB-A- 790 677

## Description

The invention relates to a method for producing a hollow structure and particularly a method for producing a closed and filled hollow structure. Furthermore, the present invention relates to a hollow structure and preferably a closed and filled hollow structure obtainable by a method according to the present invention as well as to a tool for producing such hollow structure and/or for perfomimg a respective method. More particularly, the hollow structure according to the present invention as well as the filling is at least partially flexible and/or can be brought into a flexible condition.

Such hollow structures are known in the prior art, for example in the field of face masks for delivering breathable air to a patient. In such face masks hollow cushions are used for providing contact zones for contacting the face of the user in order to avoid dents and to improve wearing comfort of the user. Furthermore, such structures are used as a sealing structure for sealing the mask interior from the exterior in the contact region, where the mask rests on a user's face.

The hollow structures known in the art are disadvantageous end that they are complicated to manufacture wherein often a number of manual manufacturing steps are necessary. Moreover respective hollow structures are either not closed, i.e., open or are closed in an ineffective and/or complicated manner.

In particular, it is known to fill a mask cushion with silicone or gel wherein the hollow structure is closed by use of a sllicone adhesive.
GB 790,677 A discloses oxygen anesthesia face masks having a hollow mask body.

The solutions known in the prior art are in particular, not easy to handle, not durable, complicated and expensive to manufacture, not suitable for automatizing, not bio-compatible as well as optically and hygienically objectionable.

Thus, it is an object underlying the present invention to provide a method for producing or manufacturing a hollow structure and particularly a filled hollow structure which overcomes the deficiencies of the prior art. More particularly, it is an object underlying the present invention to provide a method for manufacturing the hollow structure which is easy and efficient to carry out, which can be automated, and which provides for a durable as well as an optically and hygienically improved hollow structure. Moreover, it is an object underlying the present invention to provide a tool for producing a respective hollow structure as well as to provide an improved respective hollow structure as such.

The object of the present invention is fulfilled by the features of the independent claims wherein the dependent claims relate to preferred embodiments.

In particular, the present invention relates to a method for producing a filled hollow structure comprising the steps of producing an open hollow structure, e.g., a structure having a hollow, such as a pocket, or a cavity wherein at least a portion of the hollow is open towards the outside of the structure. In a next step, the hollow structure is positioned in or on a tool for holding the open hollow structure and subsequently the hollow structure is at least partially filled with a filler medium. In a next step the filled open hollow structure is closed, preferably by means of clamping jaws which in a mutual position provide a cavity into which the portions of the hollow structure to be closed extend. In this context it is to be noted that clamping jaws as referred to in the present application are not limited to perform a pivoting motion towards one another but may also or alternatively clamp via a linear motion or the like.

Subsequently, the cavity is filled with a material for closing and/or scaling the open hollow structure. Preferably, before and/or while filling the cavity with the material for closing and/or sealing the open hollow structure, pressure is applied to the cavity and/or by the clamping jaws to the opening of the hollow structure in order to securely close and/or seal the hollow structure. Preferably, this i.a. prevents the material for closing and/or sealing the open hollow structure from entering the open hollow structure. According to a preferred embodiment, the sequence of method steps is changed, e.g., in that the open hollow structure is provided with a connection means and is first closed and/or sealed, as discussed above, and subsequently at least partially filled with a filler medium through the connection means. Preferably, the connection means act as a one way valve so that they need not be closed or sealed after the filling step. Also preferably, the connection means are closed and/or sealed after the filling step. According to a further preferred embodiment, the open hollow structure is first closed and/or sealed. Subsequently a vacuum is applied to the hollow structure and then the structure is filled with a filler medium. The application of vacuum and the filling may preferably be carried out subsequently and/or simultaneously. The application of a vacuum can be considered as advantageous in case, e.g., a viscous gel is used as a filler medium.

Preferably, the open hollow structure is made of a first material and a second material is used for closing the open hollow structure. Preferably, one or both of the first and second material is an elastic material.

The hollow structure is preferably made of a flexible first material, more preferably of a plastic or synthetic material as such as an elastomer material or a material comprising elastomer components and even more preferably, the hollow structure is made of silicone such as liquid silicone rubber (LSR), or thermoplastic elastomer (TPE). In a preferred embodiment, an open hollow structure is an injection molded plastic skin, preferably made of silicone and more preferably made of liquid silicone rubber (LSR). Preferably, the hollow structure comprises a first portion to be filled by filler medium, a second portion which is constructed as a connection structure or transition portion between the first portion and a third portion which is adapted for filling and closing or scaling the hollow structure. Preferably or alternatively, the second portion is optional and the hollow structure may comprise only the first and the third portion.

The third section of the hollow structure according to a preferred embodiment, i.e., the area for filling and sealing the hollow structure is preferably designed as or preferably comprises a bi-stable membrane which may assume two positions, i.e., a first position in which the hollow structure is open for filling the structure and a second position in which the hollow structure is closed for closing and/or sealing.

The geometry of the hollow structure in the third section is preferably circular, oval, non-circular, linear etc. Preferably, the respective section comprises two opposite flattened areas which are adapted to close the hollow structure when abutting each other.

Furthermore, the hollow structure preferably comprises thin or skin-like outer walls which may have, according to preferred embodiments, a differing wall thickness and/or topography. According to a preferred embodiment, the open hollow structure may be described as having a generally tube-like form which has been put over itself resulting in a hollow structure having an inner wall and an outer wall which merge at one end of the structure via a bottom or transition wall and which is open at the other end of the structure. However, it is to be understood that the tube-like basic form as referred to above is not restricted to one diameter or a circular form but may comprise different cross sections, diameters, wall thicknesses etc. Preferably, when used in the field of face masks for delivering breathable air to a patient the hollow structure has a generally tubular ring-like form with a generally triangular cross-section, preferably particularly at its bottom or transition wall serving as a contact zone for resting on a patient's face. Preferably, when used in the field of face masks for delivering breathable air to a patient, the wall thickness varies and more preferably lies in the range of about 0,1mm to about 7mm. Preferably, the hardness of the hollow structure lies in the range from about 1 to 80 Shore A, more preferably about 20 to 60 Shore A or in the range of about 3 to 10 Shore A and preferably at about 5 Shore A or 40 Shore A. The preferred hardness particularly depends on the spring and damping properties of the material and the ability to be easily demoulded.

The filler medium which is filled into the open hollow structure preferably is or comprises a fluid, i.e., a gaseous, dispersible and/or liquid fluid, an expandable fluid, a foam, a powder and/or gel. According to preferred embodiments, a mixture of the above media is used, such as, e.g., two gels of different hardness or viscosity.

The second material used for closing the open hollow structure is preferably suitable to seal the opening of the open hollow structure in a liquid and/or gas tight manner. Preferably, the fluid comprises the same material as that from which the open hollow structure is made. According to a preferred embodiment, the material for closing the open hollow structure is silicone and more preferably liquid silicone rubber. According to a preferred embodiment, the second material used for closing the filled hollow structure is not a silicone glue or is not a silicone adhesive.

The method according to the present invention preferably further comprises the step of curing the material for closing the open hollow structure. Such curing may involve, depending on the conditions and the materials used, i.a., the addition or removal of Energy, application of temperature i.e., heat or cold, radiation, e.g., UV-light, the addition of further substances and/or time factors, i.e., waiting time etc., foaming, and/or initiation of crystallization. During curing, preferably the form or geometry of the closed or sealing area is controlled, e.g., by the clamping jaws and the respective cavity.

Preferably, the open hollow structure is held on the tool for holding the open hollow structure by the provision of a firm fit by the application of compressed air and/or vacuum. Also, the closed hollow structure is preferably removed from the tool by the use of compressed air and/or vacuum.

In a preferred additional step a vacuum is applied to the hollow before the filler medium is inserted into the hollow. The application of the vacuum to the hollow sucks air out of the hollow before the filler medium is inserted into the pocket so that an improved, e.g., void free, filling of the hollow is achieved.

In a further preferred additional step a vacuum is applied to the hollow and the filler medium after the open hollow structure has been filled with the filler medium. This step improves the optical and hygienically appearance of the filled hollow structure as well as its mechanical properties by reducing or eliminating voids occurring in the filler medium and by providing a desired shape or structure to the hollow structure.

The material for closing the open hollow structure is, according to a preferred embodiment, applied into the cavity provided by the clamping jaws under pressure. Further preferably, the clamping jaws may be heated for curing the material for closing the open hollow structure.

According to a preferred embodiment, the method according to the invention further comprises the step of subsequent coating of the hollow structure and/or the filler medium in the hollow, particularly in order to improve durability and/or optical and/or hygienically properties. According to a further preferred embodiment, the subsequent additional coating step is performed after applying a vacuum to the hollow and the filler medium, e.g., in order to provide a desired shape, and eliminate voids in the filler medium etc.

According to a preferred embodiment, the hollow structure comprises connection means for allowing to connect the hollow structure to further structures. For example, according to a preferred embodiment, the hollow structure forms part of a face mask and thus is preferably suitable to be connected with other structures, to fulfill other or additional functions, and/or to additionally form other structures in order to form such a mask.

According to a further preferred embodiment, the hollow structure comprises a connection for applying a vacuum to the hollow. This particularly allows to shape the structure if no vacuum is supplied and to fix the respective shape by application of vacuum sucking air out of the hollow. Preferably, the respective connection may be closed so that the vacuum is maintained inside the hollow structure.

The filler medium preferably is suitable to store heat and/or cold. According to a further preferred embodiment, the filler medium is suitable to harden and/or soften if certain predetermined conditions are met. Preferably, the filler medium may harden and/or soften upon a trigger signal, such as a signalization signal, or upon the application of heat or cold etc.

The present invention also provides a hollow structure particularly obtainable or obtained by a method according to the present invention as well as a tool for providing such hollow structure and for performing a method according to the present invention, respectively. The above and below discussion thus also relates to a hollow structure and a tool according to the present invention.

According to a preferred embodiment, the hollow structure constitutes a cushion to be used in face masks and preferably breathing masks in order to provide a tight and comfortable contact between mask and a user.
18. Method according to any one of the preceding items , wherein the hollow structure is a cushion for a breathing mask.
19. Hollow structure obtainable by a method according to any one of item 1 to 19.
20. Tool for producing a hollow structure according to items 20 and/or for performing a method according to any one of items 1 to 19.

The subject-matter of the present invention will further be exemplary discussed in connection with a preferred embodiment with reference to the accompanying drawings which show:
- Fig. 1: a schematic concept of a production line according to the method of the present invention;
- Fig. 2: a schematic concept of an open hollow structure;
- Fig. 3: a schematic concept of an open hollow structure placed on a tool for holding the open hollow structure;
- Fig. 4: a schematic sectional view of an open hollow structure placed on a tool for holding the open hollow structure when the filler medium is filled into the open hollow structure;
- Fig. 5: a schematic sectional view of a filled open hollow structure;
- Fig. 6: a schematic depiction of the closing or sealing area of an open hollow structure clamped by clamping means providing a cavity (Fig. 6a), wherein Fig. 6b shows a detail of Fig. 6A;
- Fig. 7: a schematic illustration of a second step of the closing step wherein Fig. 7a shows a cross section of the tool, hollow structure and clamping means wherein the cavity is filled with a material for closing the open hollow structure; wherein Fig. 7b shows a detail of Fig. 7a and wherein Fig. 7c identifies a respective detail on a closed filled hollow structure;
- Fig. 8: shows a schematic dimensional illustration of the curing step;
- Fig. 9: shows the tool for holding the open hollow structure (Fig. 9a) and the closed hollow structure removed from the tool after completion of the method (Fig. 9b);
- Fig. 10: shows a schematic sectional concept view of a filled hollow structure;
- Fig. 11: shows a schematic dimensional illustration of a hollow structure wherein the closing area is designed as a bi-stable membrane wherein Fig. 11a shows the bi- stable membrane in an open position and Fig. 11b shows a bi-stable membrane in a closed and sealed position; and
- Fig. 12: shows a schematic set up of an automated production line for carrying out the invention.

In the following the method according to the present invention will be discussed with reference to a preferred embodiment of the present invention. It is particularly to be understood that the hollow structure as referred to hereinafter may have differing forms and shapes as will also be indicated below.

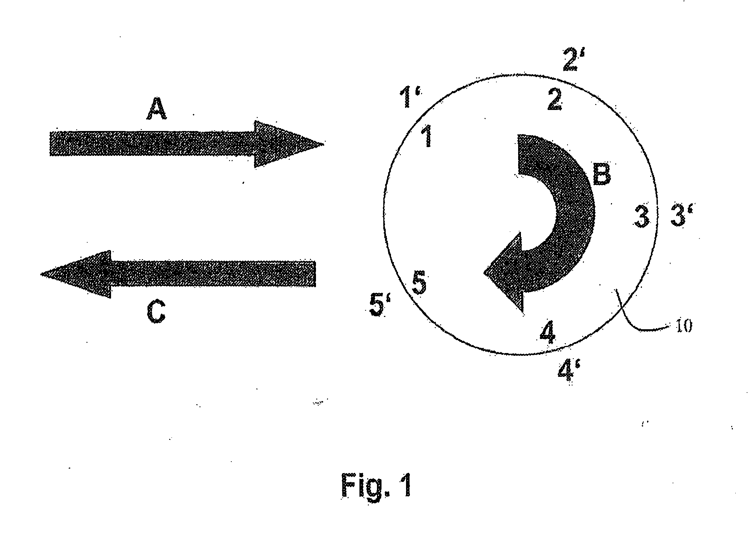

Fig. 1 shows the basic concept of the method for producing a filled hollow structure according to the present invention. In particular, arrows A, B and C show the way of the product according to the steps of the method according to the present invention.

Arrow A indicates the feeding of an open hollow structure to the tool for producing a filled hollow structure. The open hollow structure, preferably an injection molded plastic structure having thin wall thicknesses, preferably made of silicone and more preferably of liquid silicone rubber is inserted from the direction as indicated by arrow A and is positioned on a tool for holding the open hollow structure in working position 1. Preferably, working position 1 is located on a turntable 10 so that the open hollow structure is successively carried along positions 1 to 5 on table 10 wherein at each position 1 to 5 one or more method steps according to the present invention are carried out.

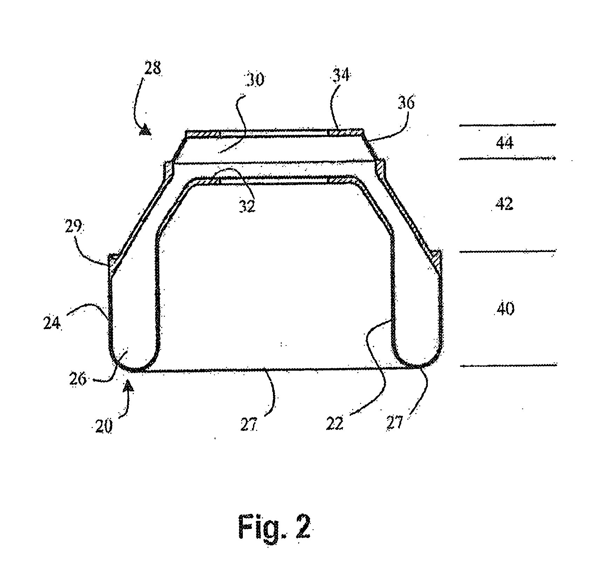

An example of an open hollow structure inserted into the tool at position 1 is shown in Fig. 2. Open hollow structure 20 as shown in Fig. 2 comprises a thin inner wall 22 and a thin outer wall 24 wherein a hollow, such as a pocket, or a cavity, 26 is formed between inner wall and outer wall 24. Inner wall 22 and outer wall 24 merge at one end of the structure via a bottom or transition wall 27. Hollow structure 20 preferably has a basically ring shaped form when viewed from the bottom in Fig. 2. Hollow structure 20 further preferably comprises a filling or sealing area 28 in which the hollow is open to the environment by opening 30. In the shown examplatory embodiment, opening 30 is a circular slot extending between a first closing area 32 and a second closing area 34. Closing areas 32 and 34 are preferably designed as flattened, basically ring shaped portions of the hollow structure which arc able to close the open hollow structure, e.g., when abutting each other in mutual contact. Preferably, closing area 32 forms part of or is directly or indirectly connected to inner wall 22 whereas closing area 34 forms part of or is directly or indirectly connected to outer wall 24. As shown in Fig. 2, closing or sealing area 28 and hollow structure 20 have a substantially circular form. However, the hollow structure and/or the closing area of the hollow structure according to the present invention is not limited to circular or substantially circular shapes but may even have other shapes as will be discussed below in further detail. According to a referred embodiment, the hollow structure and/or the closing area of the hollow structure according to the present invention have the form and/or configuration of face masks for delivering breathable air to a patient and/or of hollow cushions of such masks, such as, e.g., a basically triangular form.

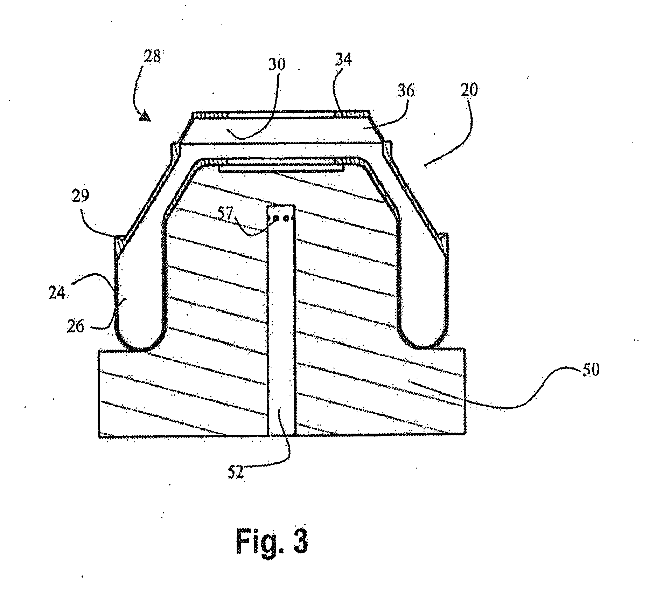

Fig. 3 shows an open hollow structure at position 1 (Fig. 1) placed on a tool 50 for holding the open hollow structure 20. Preferably, the geometry of tool 50 conforms to the geometry of the hollow structure 20 and particularly to the geometry of inner wall 22. According to a preferred embodiment, tool 50 comprises a duct 52 and nozzles or channels 57 for the application of a vacuum or compressed air to be used for holding hollow structure 20 on the tool 50 as well as for releasing or removing hollow structure 20 from the tool 50.

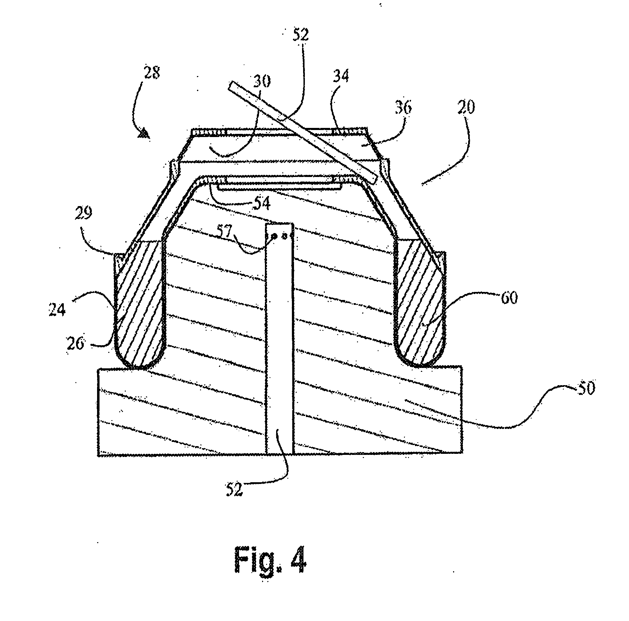

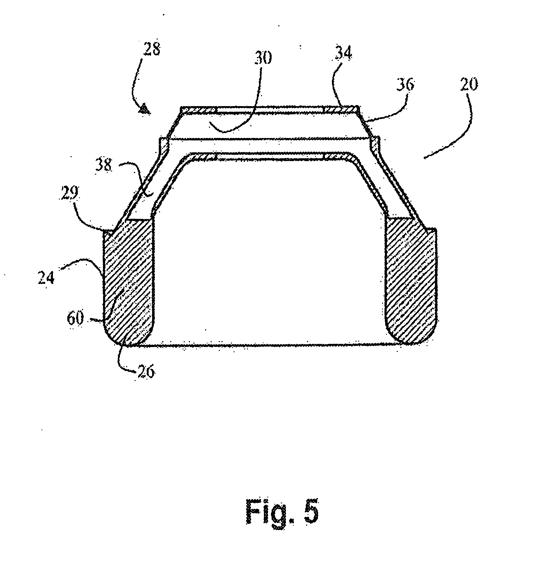

Fig. 4 shows a schematic illustration of the hollow structure 20 being at working position 2 (Fig. 1) at which the open hollow structure 20 is filled with a filler medium 60, preferably via injection pin 52. Injection pin 52 preferably is preferably connected to a supply of filler medium and is adapted to be inserted into the opening of the hollow structure to provide a fluid communication between the supply of filler medium and the hollow 26 of hollow structure 20. According to a preferred embodiment, more than one injection pin 52 may be used for filling hollow structure 20. Fig. 5 shows a cross sectional view of a hollow structure 20 according to Fig. 2 which is filled with a filler medium 60. As shown in Fig. 5, hollow 26 is completely filled with filler medium 60 wherein a remaining cavity or hollow 38 provided in a second section 42 of hollow structure 20 reaching to the closing area 28 is not filled with filler medium 60. In alternative embodiments, hollow 26 may be only partially filled with filler medium 60 or also the cavity or hollow 38 of the intermediate second section of hollow structure 20 is fully filled with filler medium 60.

In a preferred embodiment, e.g., when the filler medium is a gel or a silicone gel, the gel is inserted cold or at room temperature as a liquid and is subsequently heated to cure, as discussed further below. According to a further preferred embodiment, the gel is inserted into the hollow 26 preheated. This particularly allows a faster cure. According to a further preferred embodiment, the gel is inserted precooled into the cavity 26. This particularly allows to slow down the curing process, e.g., if the gel is curing too fast.

As a further preferred step preceding the filling of the hollow 26 with a filler medium 60 a vacuum is supplied to the hollow in order to suck air out of the hollow before the filler medium, such as a gel, is inserted.

Furthermore, the gel is preferably allowed to only cure partially for achieving desired properties of the structure.

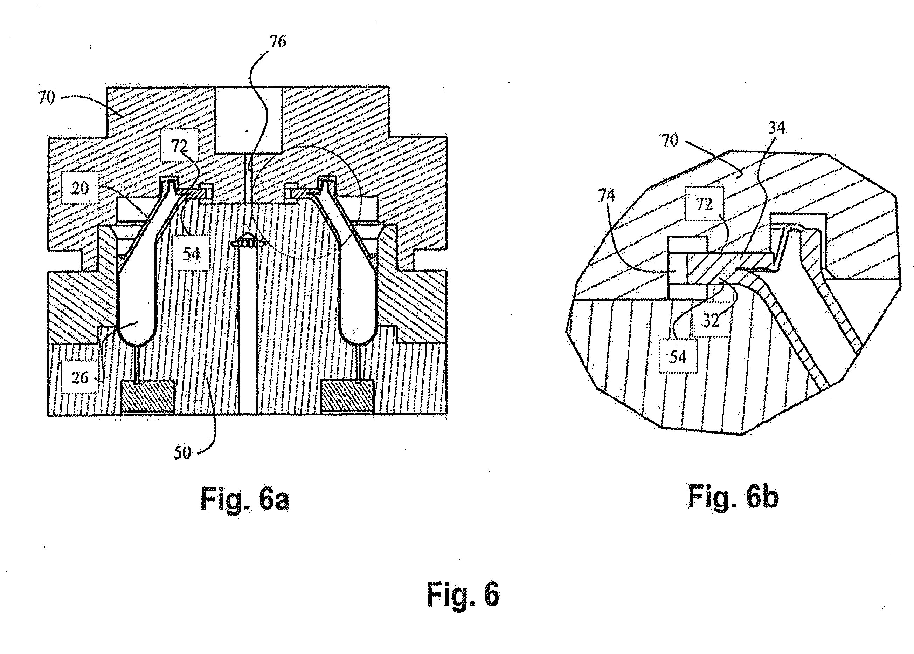

Fig. 6 shows a schematic illustration of the method carried out at working position 3 (Fig. 1). Fig. 6a shows hollow structure 20 filled or not filled with a filler medium 60 (not shown) placed on tool 50 wherein a second tool 70 is provided for closing the filled open hollow structure. In particular, tools 50 and 70 are configured to cooperate in a manner so as to close the filled open hollow structure 20. Preferably, hollow structure 20 and tool 50 are configured such that the third section 44 of hollow structure 20, i.e., the filling and the closing section 28 is located on a closing surface 54 of tool 50. Tool 70 preferably comprises a corresponding closing surface 72 which is adapted to cooperate with tool 50 and particularly with closing surface 54 of tool 50 so as to allow closing of the filled open hollow structure. Preferably, closing surfaces 54 and 72 are configured so that tool 70 when being positioned relative to tool 50 also urges closing area 34 of hollow structure 20 towards closing area 32 so that the closing surfaces 32 and 34 mutually abut each other and take a closed position, respectively. Moreover, tools 50 and 70 and particularly closing surfaces 54 and 72, respectively, provide a cavity 74 in the area of abutting closing surfaces 32 and 34 of hollow structure 20. Preferably, cavity 74 surrounds an end portion of closing areas 32 and 34 and thus of hollow structure 20. Particularly, cavity 74 is provided such that it surrounds the opening 30 or the closed opening 30 of hollow structure 20.

Tool 70 preferably comprises a channel system 76 which is in fluid communication with cavity 74 for providing a material for closing the open hollow structure to cavity 74. Preferably, channel system 76 allows a material for closing the hollow structure 20 to be filled into cavity 74, preferably under pressure. According to an alternative embodiment, channel system 76 may additionally or solely provided in tool 50.

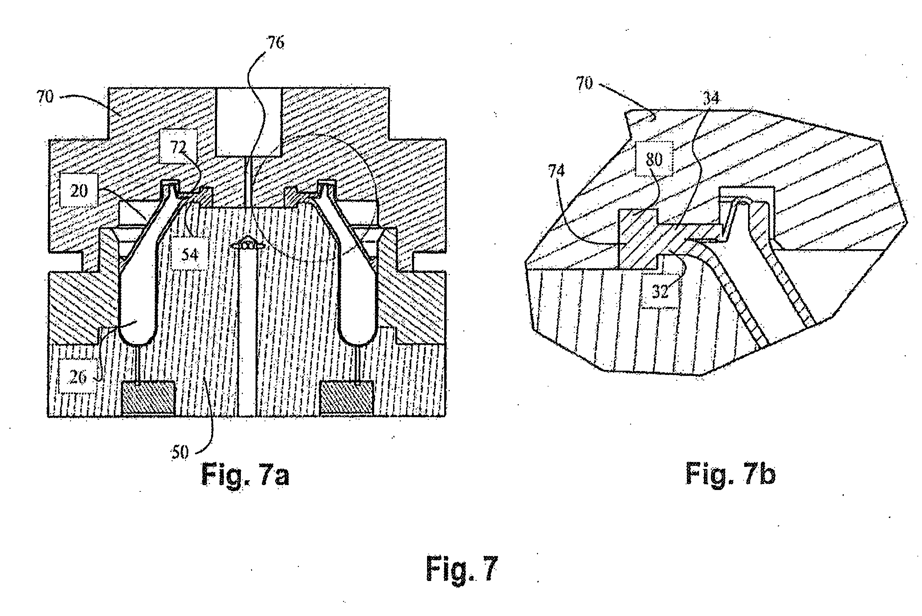

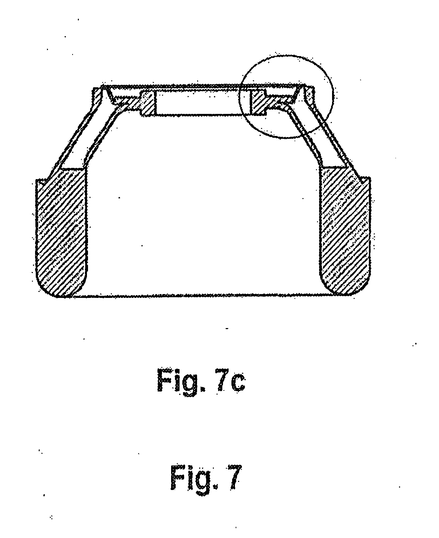

Fig. 6b shows an enlarged view of the area around the cavity 74 and the closing areas 32 and 34 of hollow structure 20. Fig. 7 shows cavity 74 being filled with a material 80 for closing the hollow structure 20 in the area of opening 30 and/or closing areas 32 and 34. Fig. 7a shows a detail of filled cavity 74 at position 3 (Fig. 1) wherein Fig. 7c shows the respective area of hollow structure 20 without tools 50 and 70, e.g. after completion of the method of the invention.

According to a preferred embodiment, tool 50 and/or tool 70 are adapted to assist curing of the material for closing the open hollow structure. According to a preferred embodiment, tools 50 and 70, respectively are heatable and/or coolable to allow the material 80 filled into cavity 74 to cure.

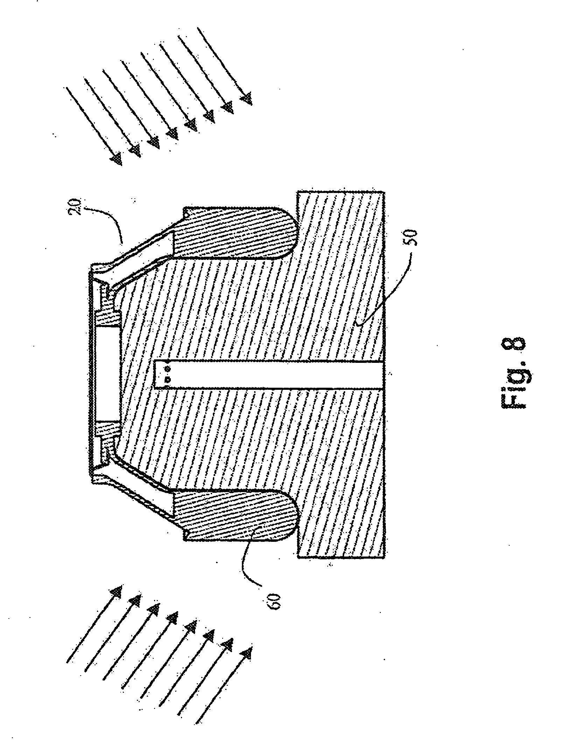

Fig. 8 shows a preferable step of the method according to the present invention at working position 4 (Fig. 1), i.e. curing of the filler medium 60 inside the closed and filled hollow structure 20. Such curing can be achieved by, e.g., the application of temperature, radiation etc. Preferably, tool 50 is heatable to assist curing of filler medium 60.

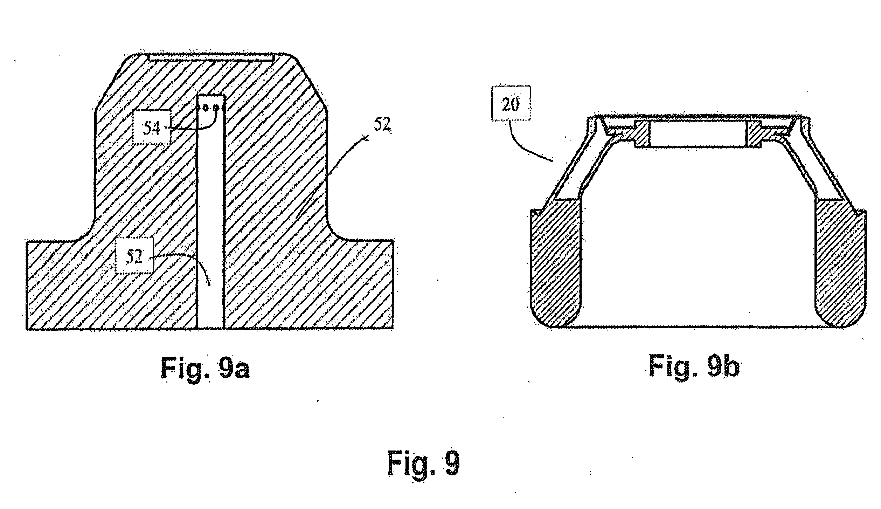

Fig. 9 shows tool 50 and hollow structure 20 after the method according to the present invention has been carried out and after hollow structure 20 has been removed from tool 50, e.g., by applying pressured air through duct 52 and nozzles 54 and/or by releasing the vacuum applied to hold hollow structure 20 on the tool 50.

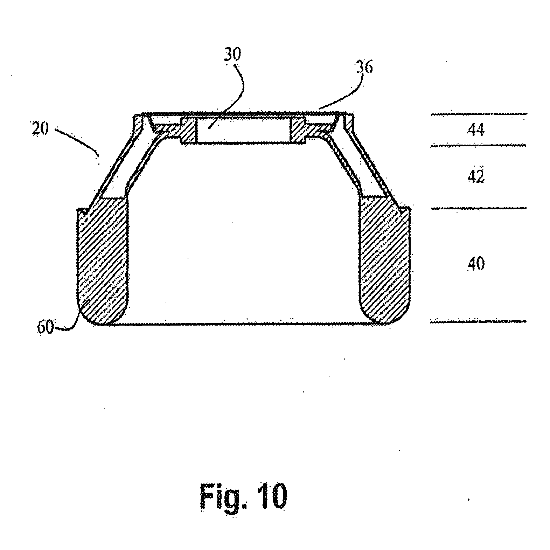

Fig. 10 shows a hollow structure 20 after having been filled with filler medium 60 and after having been closed in the area of opening 30 in the manner as described before. As is apparent from the schematic sectional view of a hollow structure 20, hollow structure 20 preferably comprises three sections. First section 40 comprises cavity 26 filled or to be filled with filler medium 60. A third section 44 comprises opening 30 and closing surfaces 32 and 34. Preferably, a second section 42 is arranged between first section 40 and third section 44 and constitutes a transition between first section 40 and third section 44. According to a preferred embodiment of the hollow structure according to the present invention, section 42 constitutes or comprises a connection structure 29 suitable for connecting hollow structure 42 to a superior structure, such as a face mask or a part of a face mask. Alternatively or additionally hollow structure 20 may comprise further means for allowing or facilitating a connection of hollow structure 22 to further structures and to allow an assembly of hollow structure 20 together with further means and structures to become a final product, such as a face mask.

As is also apparent from the schematic depiction in Fig. 10, hollow structure 20 may comprise different wall thicknesses as well as various changes in the geometry depending on the individual requirements of the desired use.

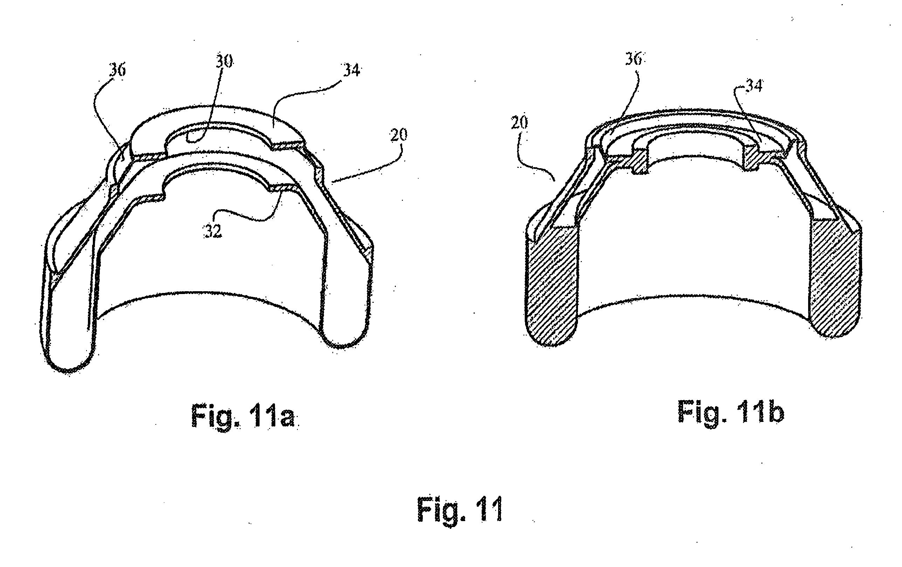

Fig. 11 shows the hollow structure in an unfilled and open state (Fig. 11a) as well as in a filled and closed or sealed state (Fig. 11b). As discussed above, hollow structure 20 comprises an opening 30, preferably arranged between section 32 and 34 for providing a closable opening 30. Sections 32 and 34 are preferably constructed as comprising mutual surfaces which when abutting each other allow the opening 30 of hollow structure 20 to be sealingly closed as discussed above with reference to Figs. 6 and 7. Preferably, hollow structure 20 comprises a bi-stable membrane 36, preferably in the area of section 34. Bi-stable membrane 36 allows section 32 to take two stable positions. In this context, the term stable refers to positions in which section 34 remains without application of additional external forces. When section 34 or bi-stable membrane 36 are in a first, opened position, hollow structure 20 is open and a filler medium 60 may be injected. Fig. 11 a shows bi-stable membrane 36 being in a first, i.e., open position. In a second, closed position, as shown in Fig. 11b, section 34 abuts section 32 so that opening 30 and thus hollow structure 20 are closed. Preferably, membrane 36 is designed in manner so that section 34 once in either the first or second stable position, strives to maintain the respective position. Even if section 34 is slightly moved from the stable position it will tend to return to the respective stable position. Only, if section 34 travels a defined way from a stable position towards the other stable position it will spring or snap to the other stable position after having been moved over a point of no return. These effects are achieved by the provision of bi-stable membrane 36 which is designed accordingly by varying its geometry and wall thickness depending on the overall geometry of opening 30, sections 32 and particularly 34.

At working position 5 as indicated in Fig. 1, the filled enclosed hollow structure 20 is removed from the table 10, see arrow C. Subsequently, further method steps may be applied.

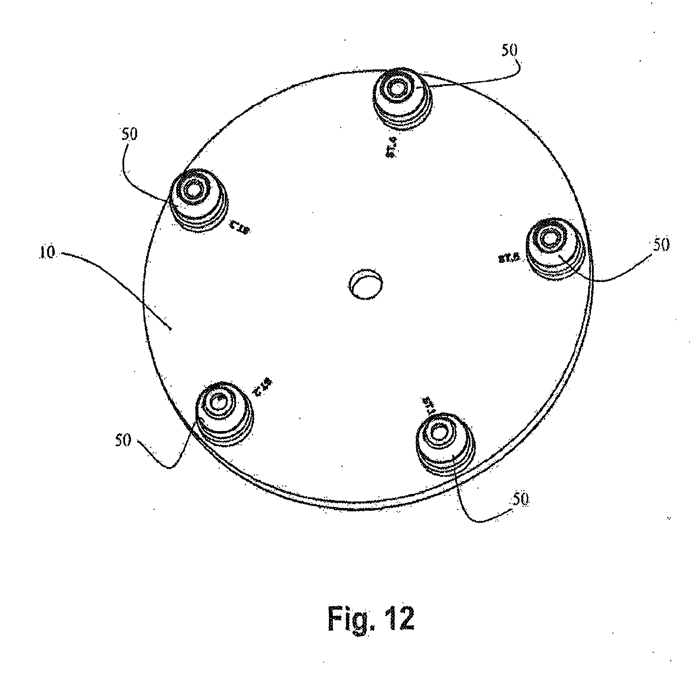

Fig. 12 shows a schematic three-dimensional view of a table 10 with five working positions at which tools 50 are provided. A filled hollow structure 20 positioned on a tool 50 at working position I may thus simply undergo the respective method treatments as discussed above by turning table 10 in the direction of arrow B as indicated in Fig. 1 so that the respective hollow structure 20 moves from a first working position to a second position etc. Further means for carrying out the respective method steps are provided at working stations 1', 2', 3', 4', 5' outside the table 10 and apply the same method steps to different hollow structures 20 passing the working station after each cycle. Accordingly, referring to Fig. 1, a hollow structure is placed on tool 50 at position I and working station 1' is then moved by turning turntable 10 to working station 2'. At the same time, working position 5 moves to working station 1' and a new hollow structure is placed onto respective tool 50 at working position 5. At working station 2' filling of the filler material is carried out. In the next cycle, the hollow structure at position I moves from working station 2' to working station 3' where the closing of the hollow structure is performed as discussed above. At the same time, hollow structure 20 positioned on working position 5 moves from working station 1' to working station 2'. After the further method steps have been performed, here the curing at working station 4' and removing the finished hollow structure at working station 5' working position 1 again arrives at working station 1' and a new hollow structure is inserted. It is to be noted, as discussed above, that step 4 regarding curing of the inserted filler material is an optional step of the method according to the present invention.

As discussed above, the above description as well as the figures relate to an exemplary schematic example in order to more clearly explain the method and product underlying the present invention. However, the respective method as well as product and tool and in particular their geometry are not considered as being restricted by the above example. According to further preferred embodiments, a preferred hollow structure, as e.g. depicted in Figs. 9c and 11 does not have a circular cross section in a top and/or bottom view but may comprise different forms. Preferably, a respective hollow structure has a substantially triangular form when viewed from the top and/or bottom. However, the hollow structure according to the present invention may also comprise further symmetrical and not symmetrical forms in a top and/or bottom view such as a rectangular, elliptical, round, ringshaped and/or linear etc.

As discussed earlier, the filler medium may be a fluid such as a gaseous and/or liquid medium, a gel, a powder, beats or pellets, a foam or a foamable medium etc. Preferably, the filler medium allows the hollow structure to yield or react resiliently upon application of external pressure and provides a soft and comfortable deformable appearance. Preferably, softness or hardness of the filler medium or the filled hollow structure may be adapted according to the requirements of the desired use either during production of the filled hollow structure or after production and prior to use. This can be established by either adjusting the geometry and wall thicknesses of the hollow structure allowing to provide a general support of the filler medium, the degree up to which the hollow is filled with the filler medium, the filler medium itself as well as a combination of these factors. For example, referring to Fig. 2, According to a preferred embodiment the hollow structure may comprise different wall thicknesses, structures for fulfilling additional objects and/or the like.

Preferably, the hollow structure comprises a connection for applying a vacuum to the hollow thereby allowing to deform the hollow structure so that a desired shape is achieved and subsequently applying a vacuum which is maintained in the hollow after closure of the respective connection so that the shape of the hollow structure is maintained. Further, a desired shape or individual shape may be formed and fixed by, e.g., a curing process of the filler medium. Preferably, the filler medium and thus the filled hollow structure is still deformable and soft to a certain degree even after fixation of a desired shape as discussed above.

According to a preferred embodiment, the hollow structure is made of a silicone material, preferably of liquid silicone rubber wherein the filler medium is also a silicone material, preferably liquid silicone rubber in a more or less liquid aggregate state such as gel which may be achieved by a partial curing or adding additives.

## Claims

1. Method for producing a filled hollow structure (20) constituting a cushion for a breathing mask comprising the steps:
producing an open hollow structure (20) of a first material
positioning the open hollow structure on a tool (10) for holding the open hollow structure (20);
filling the open hollow structure (20) with a filler medium;
closing the filled open hollow structure (20) by means of a second material.

2. Method according to claim 1, the first hollow structure including a inner wall (22) and an outer wall (24) between which a hollow area (26) is formed, the hollow structure having an opening (30), the opening having a first closing area (32) connected to the inner wall (22) and a second closing area (34) connected to the outer wall (24), wherein the structure is closed by means of urging the second closing area towards the first closing area so that the first and second closing areas are mutually abutted together.

3. Method according to claim 2, comprising the further step of sealingly closing the mutually abutted first and second closing areas with the second material.

4. Method according to claim 2 or 3, wherein the second closing area (34) further comprises a bi-stable membrane (36) that is moved from first opened position to a second closed position during the step of closing the filled hollow structure.

5. Method according to any one of claims 1-4, wherein the filling step includes application of pressure, vacuum, cold and/or heat.

6. Method according to any one of claims 1-5, wherein the first material is silicone and/or wherein the second material is silicone, liquid silicone rubber (LSR) or thermoplastic elastomer (TPE) and, preferably, wherein the second material is not a silicone glue and/or is not a silicone adhesive.

7. Method according to any one of the preceding claims, wherein the filler medium is a fluid, gas, liquid, foam, expandable fluid, powder, gel and/or two gels of different hardness or viscosity; and/or wherein the filler medium preferably is a silicone gel, a silicone material, or a liquid silicone rubber.

8. Method according to any one of the preceding claims, the hollow structure being a structure with a pocket or cavity, preferably having a tubular form, and/or preferably comprising skin-like outer walls which preferably have a differing wall-thickness and/or topography.

9. Method according to any one of the preceding claims, wherein the open hollow structure is filled via an injection pin.

10. Method according to any one of the preceding claims, wherein the hollow structure is completely or partially filled with the filler medium.

11. Method according to any one of the preceding claims, wherein the hollow structure is provided with connection means and/or wherein the hollow structure comprises a connection for applying a vacuum or pressure.

12. Method according to any one of the preceding claims, wherein the filler medium stores heat and/or cold, and/or wherein the filler medium is suitable to harden and/or soften upon a trigger signal, such as a crystallization signal or the application of heat or cold.

13. Tool for performing a method according to any one of claims 1 to 12, the tool comprising a first tool part (50) and a second tool part (70) which are configured to cooperate in a manner so as to close the filled open hollow structure (20), the first tool part (50) comprising a closing surface (54) and the second tool part (70) comprising a corresponding closing surface (72) adapted to cooperate with first tool part (50) to provide a cavity (74) so as to allow closing the filled open structure by providing the second material to cavity (74) via a channel system (76) of second tool part (70).

14. A hollow cushion structure adapted to form part of a face mask, comprising
an inner wall (22);
an outer wall (24);
a hollow area (26) formed between the inner and outer wall adapted to be filled by a first material,
an opening (30), preferably having a first closing area (32) connected to the inner wall (22) and a second closing area (34) connected to the outer wall (24);
wherein, preferably, the second closing area (34) moves between a first, opened position in which the second closing area (34) is spaced from the first closing area (32), and a second closed position in which the second closing area (34) abuts the first closing area (32) to close the opening;
the hollow area may be filled with a first material when in the opened position;
and wherein the hollow cushion structure is adapted to be closed by means of a second material.

15. The hollow cushion structure according to claim 14 further comprising a bi-stable membrane (36) attached to the second closing area to facilitate the movement between the opened position and the closed position.

## Patentansprüche

1. Verfahren zur Herstellung einer gefüllten Hohlstruktur (20), die ein Polster für eine Atemmaske bildet, mit den folgenden Schritten:
Herstellen einer offenen Hohlstruktur (20) aus einem ersten Material;
Positionieren der offenen Hohlstruktur auf einem Werkzeug (10) zum Halten der offenen Hohlstruktur (20);
Füllen der offenen Hohlstruktur (20) mit einem Füllmittel;
Verschließen der gefüllten offenen Hohlstruktur (20) mittels eines zweiten Materials.

2. Verfahren-nach Anspruch 1, wobei die erste Hohlstruktur eine Innenwand (22) und eine Außenwand (24) aufweist, zwischen denen ein hohler Bereich (26) ausgebildet ist, wobei die Hohlstruktur eine Öffnung (30) hat, wobei die Öffnung einen ersten Schließbereich (32), der mit der Innenwand (22) verbunden ist, und einen zweiten Schließbereich (34) hat, der mit der Außenwand (24) verbunden ist, wobei die Struktur geschlossen wird, indem der zweite Schließbereich in Richtung des ersten Schließbereichs gedrückt wird, so dass der erste und zweite Schließbereich gegenseitig aneinander anliegen.

3. Verfahren nach Anspruch 2 mit dem folgenden weiteren Schritt: dichtes Verschließen der gegenseitig aneinander anliegenden ersten und zweiten Schließbereiche mit dem zweiten Material.

4. Verfahren nach Anspruch 2 oder 3, wobei der zweite Schließbereich (34) ferner eine bistabile Membran (36) aufweist, die während des Schritts des Schließens der gefüllten Hohlstruktur aus einer ersten, geöffneten Position in eine zweite, geschlossene Position bewegt wird.

5. Verfahren nach einem der Ansprüche 1 bis 4, wobei der Füllschritt die Anwendung von Druck, Vakuum, Kälte und/oder Wärme umfasst.

6. Verfahren nach einem der Ansprüche 1 bis 5, wobei das erste Material Silikon ist und/oder wobei das zweite Material Silikon, Flüssigsilikonkautschuk (LSR) oder thermoplastisches Elastomer (TPE) ist, und wobei vorzugsweise das zweite Material kein Silikonleim und/oder kein Silikonkleber ist.

7. Verfahren nach einem der vorhergehenden Ansprüche, wobei das Füllmittel ein Fluid, Gas, Flüssigkeit, Schaum, verschäumbares bzw. ausdehnbares Fluid, Pulver, Gel und/oder zwei Gele unterschiedlicher Härte oder Viskosität ist; und/oder wobei das Füllmittel vorzugsweise ein Silikongel, ein Silikonmaterial oder ein Flüssigsilikonkautschuk ist.

8. Verfahren nach einem der vorhergehenden Ansprüche, wobei die Hohlstruktur eine Struktur mit einer Tasche oder einem Hohlraum ist, vorzugsweise röhrenförmig und/oder vorzugsweise mit hüllenartigen bzw. hautartigen Außenwänden, die vorzugsweise eine unterschiedliche Wanddicke und/oder Topografie haben.

9. Verfahren nach einem der vorhergehenden Ansprüche, wobei die offene Hohlstruktur über eine Injektionsnadel gefüllt wird.

10. Verfahren nach einem der vorhergehenden Ansprüche, wobei die Hohlstruktur vollständig oder teilweise mit dem Füllmittel gefüllt wird.

11. Verfahren nach einem der vorhergehenden Ansprüche, wobei die Hohlstruktur mit einer Verbindungseinrichtung versehen ist und/oder wobei die Hohlstruktur eine Verbindung zur Anwendung eines Vakuums oder Drucks aufweist.

12. Verfahren nach einem der vorhergehenden Ansprüche, wobei das Füllmittel Wärme und/oder Kälte speichert und/oder wobei das Füllmittel geeignet ist, auf ein Auslösersignal hin, wie etwa ein Kristallisationssignal oder die Anwendung von Wärme oder Kälte, hart und/oder weich zu werden.

13. Werkzeug zur Durchführung eines Verfahrens nach einem der Ansprüche 1 bis 12, wobei das Werkzeug ein erstes Werkzeugteil (50) und ein zweites Werkzeugteil (70) aufweist, die dafür konfiguriert sind, entsprechend zusammenzuwirken, um die gefüllte offene Hohlstruktur (20) zu schließen, wobei das erste Werkzeugteil (50) eine Schließfläche (54) aufweist und das zweite Werkzeugteil (70) eine entsprechende Schließfläche (72) aufweist, die dafür eingerichtet ist, mit dem ersten Werkzeugteil (50) zusammenzuwirken, um einen Hohlraum (74) bereitzustellen, so dass die gefüllte offene Hohlstruktur dadurch geschlossen werden kann, dass das zweite Material über ein Kanalsystem (76) des zweiten Werkzeugteils (70) in den Hohlraum (74) verbracht wird.

14. Hohlpolsterstruktur, die dafür eingerichtet ist, einen Teil einer Gesichtsmaske zu bilden, mit
einer Innenwand (22);
einer Außenwand (24);
einem hohlen Bereich (26), der zwischen der Innen- und der Außenwand ausgebildet ist, um mit einem ersten Material gefüllt zu werden,
einer Öffnung (30) vorzugsweise mit einem ersten Schließbereich (32), der mit der Innenwand (22) verbunden ist, und einem zweiten Schließbereich (34), der mit der Außenwand (24) verbunden ist;
wobei vorzugsweise der zweite Schließbereich (34) sich bewegt zwischen einer ersten, offenen Position, in der der zweite Schließbereich (34) von dem ersten Schließbereich (32) beabstandet ist, und einer zweiten, geschlossenen Position, in der der zweite Schließbereich (34) am ersten Schließbereich (32) anliegt, um die Öffnung zu schließen;
der hohle Bereich mit einem ersten Material gefüllt werden kann, wenn er in der geöffneten Position ist;
und wobei die Hohlpolsterstruktur dafür eingerichtet ist, mittels eines zweiten Materials geschlossen zu werden.

15. Hohlpolsterstruktur nach Anspruch 14, ferner mit einer bistabilen Membran (36), die an dem zweiten Schließbereich angebracht ist, um die Bewegung zwischen der geöffneten und der geschlossenen Position zu erleichtern.

## Revendications

1. Procédé de production d'une structure creuse comblée (20) formant un coussin pour un masque respiratoire, comprenant les étapes :
de réalisation d'une structure creuse ouverte (20) en un premier matériau ;
de positionnement de la structure creuse ouverte sur un outil (10) pour maintenir la structure creuse ouverte (20) ;
de comblement de la structure creuse ouverte (20) par un agent de charge ;
de scellage de la structure creuse ouverte comblée (20) au moyen d'un deuxième matériau.

2. Procédé selon la revendication 1, où la première structure creuse comporte une paroi intérieure (22) et une paroi extérieure (24) entre lesquelles est.formée une zone creuse (26), la structure creuse présentant une ouverture (30), ladite ouverture présentant une première zone de scellage (32) reliée à la paroi intérieure (22) et une deuxième zone de scellage (34) reliée à la paroi extérieure (24), la structure étant fermée en repoussant la deuxième zone de scellage vers la première zone de scellage de manière à rendre adjacentes l'une à l'autre la première et la deuxième zones de scellage.

3. Procédé selon la revendication 2, comprenant l'autre étape de scellage par le deuxième matériau de la première et de la deuxième zones de scellage adjacentes l'une à l'autre.

4. Procédé selon la revendication 2 ou la revendication 3, où la deuxième zone de scellage (34) comporte en outre une membrane bi-stable (36) déplacée d'une première position d'ouverture vers une deuxième position de fermeture pendant l'étape de scellage de la structure creuse comblée.

5. Procédé selon l'une des revendications 1 à 4, où l'étape de remplissage comprend l'application de pression, de vide, de froid et/ou de chaleur.

6. Procédé selon l'une des revendications 1 à 5, où le premier matériau est du silicone et/ou où le deuxième matériau est du silicone, du caoutchouc silicone liquide (LSR) ou un élastomère thermoplastique (TPE) et où, préférentiellement, le deuxième matériau n'est pas une colle silicone et/ou n'est pas un adhésif silicone.

7. Procédé selon l'une des revendications précédentes, où l'agent de charge est un fluide, un gaz, un liquide, une mousse, un fluide expansible, une poudre, un gel et/ou deux gels de duretés ou viscosités différentes ; et/ou où l'agent de charge est préférentiellement un gel silicone, un matériau silicone, ou un caoutchouc silicone liquide.

8. Procédé selon l'une des revendications précédentes, où la structure creuse est une structure avec une poche ou cavité, préférentiellement de forme tubulaire, et/ou comprenant préférentiellement des parois extérieures en forme de peau présentant préférentiellement des épaisseurs et/ou des topographies différentes.

9. Procédé selon l'une des revendications précédentes, où la structure creuse ouverte est comblée au moyen d'une filière d'injection.

10. Procédé selon l'une des revendications précédentes, où la structure creuse est complètement ou partiellement comblée par l'agent de charge.

11. Procédé selon l'une des revendications précédentes, où la structure creuse est prévue avec un moyen de connexion et/ou où la structure creuse comporte une connexion pour l'application de vide ou de pression.

12. Procédé selon l'une des revendications précédentes, où l'agent de charge conserve la chaleur et/ou le froid, et/ou où l'agent de charge est adapté pour durcir et/ou ramollir à un signal de déclenchement tel qu'un signal de cristallisation ou par application de chaleur ou de froid.

13. Outil pour l'exécution d'un procédé selon l'une des revendications 1 à 12, ledit outil comprenant une première pièce d'outil (50) et une deuxième pièce d'outil (70) prévues pour coopérer de manière à sceller la structure creuse ouverte comblée (20), la première pièce d'outil (50) présentant une surface de scellage (54) et la deuxième pièce d'outil (70) présentant une surface de scellage (72) correspondante apte à coopérer avec la première pièce d'outil (50) pour obtenir une cavité (74) permettant le scellage de la structure creuse ouverte par refoulement du deuxième matériau dans la cavité (74) par un système de canal (76) de la deuxième pièce d'outil (70).

14. Structure de coussin creuse prévue pour faire partie d'un masque facial, comprenant
une paroi intérieure (22) ;
une paroi extérieure (24) ;
une zone creuse (26) formée entre la paroi intérieure et la paroi extérieure, apte à être comblée par un premier matériau,
une ouverture (30), présentant préférentiellement une première zone de scellage (32) reliée à la paroi intérieure (22) et une deuxième zone de scellage (34) reliée à la paroi extérieure (24) ;
la deuxième zone de scellage (34) se déplaçant préférentiellement entre une première position d'ouverture où la deuxième zone de scellage (34) est espacée de la première zone de scellage (32), et une deuxième position de fermeture où la deuxième zone de scellage (34) est adjacente à la première zone de scellage (32) pour fermer l'ouverture ; la zone creuse pouvant être comblée avec un premier matériau en position d'ouverture ; et ladite structure de coussin creuse étant prévue pour être scellée avec un deuxième matériau.

15. Structure de coussin creuse selon la revendication 14, comprenant en outre une membrane bi-stable (36) fixée à la deuxième zone de scellage pour faciliter le déplacement entre la position d'ouverture et la position de fermeture.
